(19) Europäisches Patentamt
European Patent Office
Office européen des brevets

(11) Numéro de publication : **0 024 983**
**B1**

(12)

# FASCICULE DE BREVET EUROPÉEN

(45) Date de publication du fascicule du brevet : **25.07.84**

(51) Int. Cl.³ : **C 07 J 41/00**

(21) Numéro de dépôt : **80401204.5**

(22) Date de dépôt : **22.08.80**

(54) **Dérivés d'amino-14 stéroides et procédé pour leur préparation.**

(30) Priorité : **31.08.79 FR 7921844**

(43) Date de publication de la demande :
**11.03.81 Bulletin 81/10**

(45) Mention de la délivrance du brevet :
**25.07.84 Bulletin 84/30**

(84) Etats contractants désignés :
**AT BE CH DE FR GB IT LI LU NL SE**

(56) Documents cités :
**BULLETIN DE LA SOCIETE CHIMIQUE DE FRANCE. no. 9-10. septembre-octobre 1976. Paris FR A. ASTIER et al.: "Alcaloides stéroidiques CLXXIV synthèse d'azides tertiaires IV. Azido et amino-14 prégnanes", pages 1581-1582**
**TETRAHEDRON, vol. 34 (10) 1978 Pergamon Press Oxford GB A. ASTIER et al.: « Alcaloides stéroidiques - CLXXVIII Synthèse d'azides tertiaires - VIII Synthèse d'azido et d'amino - 14 beta-cardénolides », pages 1487-1491**
**Le dossier contient des informations techniques présentées postérieurement au dépôt de la demande et ne figurant pas dans le présent fascicule.**

(73) Titulaire : **ETABLISSEMENTS NATIVELLE S.A.**
**27, rue de la Procession**
**F-75015 Paris (FR)**

(72) Inventeur : **Jarreau, François-Xavier**
**5 rue Louis Hervé**
**F-78000 Versailles (FR)**
Inventeur : **Koenig, Jean-Jacques**
**31 rue du Panorama**
**F-77670 Vernou La Celle sur Seine (FR)**

(74) Mandataire : **L'Helgoualch, Jean et al**
**OFFICE PICARD 134 Boulevard de Clichy**
**F-75018 Paris (FR)**

**EP 0 024 983 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention, réalisée dans les laboratoires du CERES — Centre Européen de Recherche Scientifique — concerne de nouveaux composés dérivés de stéroïdes, et plus particulièrement des amino-14 stéroïdes substitués en 17 par un groupe fonctionnel, ainsi qu'un procédé pour leur préparation.

On sait que l'on peut transformer une fonction alcool tertiaire en azide, par action d'acide azothydrique en présence d'éthérate de trifluorure de bore en milieu benzénique. Par exemple la synthèse d'azido-14 pregnanes et d'amino-14 pregnanes par un tel procédé est décrite par Astier et al. dans Bull. Soc. Chim. 1976, 1581.

La présente invention a pour objet de nouveaux composés dérivés d'amino-14 stéroïdes, susceptibles d'être utilisés comme médicaments, ou comme intermédiaires dans la fabrication de composés utiles comme médicaments.

Les nouveaux composés conformes à la présente invention peuvent être représentés par la formule générale (I) suivante

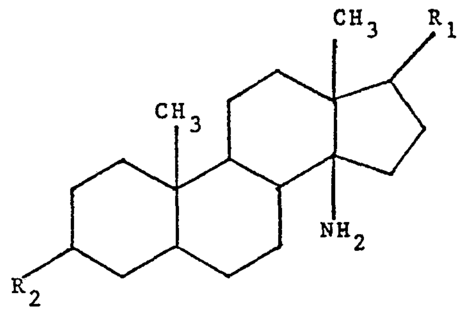

(I)

dans laquelle $R_1$ représente un groupe hydroxy, hydroxyalkyle, alcoxy, acyloxy, carboxy, acyloxyalkyle, ou carbalcoxy, ou un atome d'hydrogène, et $R_2$ représente un groupe hydroxy, alcoxy, acyloxy ou amino.

Les composés de formule générale (I) ci-dessus peuvent être préparés à partir des alcools de formule générale (II) :

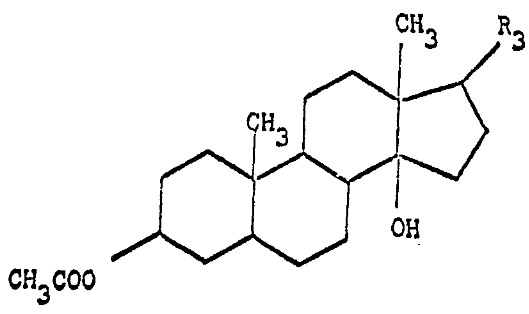

(II)

dans laquelle $R_3$ est un groupe carbalcoxy ou acyloxy, ou un atome d'hydrogène, par action de l'acide azothydrique en présence d'éthérate de trifluorure de bore dans un solvant approprié pour former un dérivé azido-14 que l'on réduit ensuite pour former un amido-14 stéroïde de formule générale (I).

Les produits de départ de formule générale (II) et en particulier l'acétoxy-3β hydroxy-14β androstane et le diacétoxy-3β, 17β hydroxy-14β androstane peuvent être aisément obtenus à partir de l'hydroxy-3 androstanone-17 préparée selon la méthode décrite par N. Danielli et al. Tetrahedron, 1967, *23*, 715 ; de même, l'acétoxy-3β hydroxy-14β 5β-étianate de méthyle peut être aisément obtenu comme décrit par F. Hunziker et al. Helv. Chim. Acta 1945, *28*, 1472.

Suivant le procédé conforme à la présente invention, on traite l'alcool de formule générale (II) par de l'acide azothydrique en excès, dans un solvant tel que le benzène ou le toluène, en présence d'éthérate de trifluorure de bore. La réaction s'effectue avantageusement à température ambiante.

Dans ces conditions, l'alcool est transformé en azide correspondant, représenté par la formule générale (II) où le groupe OH en 14 est remplacé par un groupe $N_3$.

Après extraction de l'azide, et purification le cas échéant, on effectue une réaction de réduction pour former l'amino-14 stéroïde de formule générale (I). La réduction peut être effectuée par hydrogénation de l'azide en présence d'un catalyseur tel que le catalyseur de Lindlar. On peut également utiliser un hydrure tel que l'hydrure de lithium-aluminium comme réducteur dans un solvant tel que le tétrahydrofuranne ou un éther. La réduction de l'azide en amine peut également être réalisée au moyen de réducteurs usuels appropriés, tels que des réducteurs soufrés, des réducteurs métalliques, etc. Le choix de la méthode de réduction permet d'orienter la réduction préférentiellement vers les amino-14 stéroïdes substitués par un

groupe hydroxy ou hydroxyalkyle, dans le cas de la méthode à l'hydrure de lithium-aluminium, ou vers les autres dérivés substitués par un groupe acyloxy ou carbalcoxy, en utilisant la première méthode. De plus, le groupe carbalcoxy peut être aisément transformé en groupe carboxyle, par les procédés usuels.

Si on le désire, les composés de formule générale (I) dans laquelle $R_1$ représente un groupe acyloxyalkyle (par exemple un groupe acétoxyméthyle ou acétoxyéthyle) et $R_2$ représente un groupe hydroxy, peuvent être transformés aisément en composés correspondants représentés par la formule générale (I) dans laquelle $R_2$ est un groupe amino et $R_1$ conserve la même définition ou représente un groupe hydroxyalkyle. Cette transformation peut s'effectuer par des méthodes connues, et par exemple en oxydant le groupe hydroxy représenté par $R_2$, en groupe oxo, par exemple au moyen du réactif de Jones, puis en ajoutant de l'acide formique, en faisant ensuite agir de l'hydroxylamine et en effectuant une réduction par un amalgame d'aluminium. Le groupe acyloxyalkyle représenté par $R_1$ peut être transformé en groupe hydroxyalkyle par simple hydrolyse en milieu basique.

Parmi les composés de formule générale (I), l'invention concerne de préférence ceux pour lesquels $R_1$ est un groupe hydroxy, hydroxyméthyle, acétoxy ou carbométhoxy ou un atome d'hydrogène, et $R_2$ un groupe hydroxy ou acétoxy.

L'invention concerne également les sels des amino-14 stéroïdes de formule générale (I), obtenus de façon usuelle, par réaction, en proportions sensiblement stœchiométriques, avec un acide approprié, tel que l'acide chlorhydrique, sulfurique, acétique, phosphorique, oxalique, lactique, tartrique, maléique, etc., de préférence dans un solvant compatible.

Les exemples donnés ci-après illustrent l'invention plus en détail, sans en limiter la portée.

## Exemple 1

Acétoxy-3β azido-14β étianate de méthyle.

Dans un ballon de 3 l on place 31,2 g d'acétoxy-3β hydroxy-14β 5β-étianate de méthyle préparé par la méthode de F. Hunziker et al. (Helv. Chim. Acta, 1945, *28*, 1472), en solution dans 100 ml de benzène. On ajoute progressivement 1 l d'une solution benzénique d'acide azothydrique, de concentration sensiblement molaire, puis 80 ml d'éthérate de trifluorure de bore, à température ambiante. La réaction s'effectue en maintenant le milieu sous agitation pendant 15 mn.

On noie le milieu par de l'ammoniaque glacée, on lave le phase benzénique par une solution de bicarbonate de sodium, puis on effectue une extraction par le chlorure de méthylène.

Après purification par distillation et recristallisation dans l'acétone on obtient 16,1 g d'acétoxy-3β azido-14β étianate de méthyle (rendement 50 %).

Point de fusion F = 204 – 205 °C (acétone)

Spectre de RMN ($CDCl_3$) δ = 0,96 (3H, s) 1,00 (3H, s) 2,03 (3H, s) 0,7 à 2,4 (21H) 2,53 (1H, t, J = 5) 3,68 (3H, s) 5,07 (1H) ppm

Spectre IR : $\nu$ = 2 110, 2 095, 1 725 $cm^{-1}$ (Nujol)

## Exemple 2

Diacétoxy-3β, 17βazido-14β 5β-androstane.

On l'obtient par le même procédé que dans l'exemple 1, à partir du diacétoxy-3β, 17β hydroxy-14β 5β-androstane obtenu par réduction de la cétone correspondante préparée par la méthode de N. Danielli et al. (Tétrahédron 1967, *23*, 715). Le rendement de la réaction est pratiquement quantitatif.

Spectre de RMN ($CDCl_3$) δ = 0,98 (3H, s) 1,03 (3H, s) 0,7 à 2,6 (21H) 2,05 (6H, s) 5,03 (1H) 5,06 (1H) ppm

## Exemple 3

Acétoxy-3β azido-14β androstane.

On procède comme dans l'exemple 1 en traitant l'acétoxy-3β hydroxy-14β androstane par une solution benzénique d'acide azothydrique et de l'éthérate de trifluorure de bore.

On obtient un résidu huileux purifié par chromatographie sur colonne. Après purification on obtient avec un rendement de 60 % des cristaux blancs d'acétoxy-3β azido-14β androstane.

Spectre IR $\nu$ = 2 105, 2 080, 1 725, 1 230 et 1 020 $cm^{-1}$ (Nujol)

Spectre de RMN ($CDCl_3$) = 0,96 (3H, s) 1,01 (3H, s) 0,8 à 2,8 (23H) 2,04 (3H, s) 5,08 (1H) ppm

## Exemple 4

Hydroxy-3β amino-14β androstane.

On traite 1 g d'acétoxy-3β azido-14β androstane obtenu comme indiqué dans l'exemple 3, en solution

dans 100 ml de tétrahydrofuranne, par 7 grammes d'hydrure de lithium-aluminium. On noie par de l'acétate d'éthyle, on filtre et on distille le filtrat. Le résidu est lavé par de l'acétate d'éthyle puis extrait par de l'acide chlorhydrique 0,5 N. Après alcalinisation des phases aqueuses par l'ammoniaque et une nouvelle extraction suivie d'un lavage, d'un séchage et d'une distillation, on obtient 0,7 g d'hydroxy-3β amino-14β androstane (rendement 78 %).

Spectre IR : $\nu$ = 2 600 à 3 600, 1 585 et 1 030 $cm^{-1}$ (film $CHCl_3$)

Spectre de RMN ($CDCl_3$) δ = 0,93 (6H, s) 0,7 à 2,3 (23H) 1,6 (3H mobiles) 4,10 (1K) ppm

CCM ($CHCl_3$—MeOH—$NH_4OH$ ; 90-10-1) Rf = 0,4

Par les techniques usuelles, on prépare le chlorhydrate correspondant dont le point de fusion est F > 260 °C (déc.)

Exemple 5

Acétoxy-3β amino-14β étianate de méthyle.

A 0,5 g de l'azide obtenu comme indiqué dans l'exemple 1, en solution dans 100 ml d'éthanol, on ajoute 0,25 g de palladium à 5 % sur carbonate de calcium (catalyseur de Lindlard). La réaction de réduction s'effectue pendant 20 h, sous agitation, en atmosphère d'hydrogène.

Après filtration et distillation, le résidu est recristallisé dans l'éthanol pour procurer 0,3 g de cristaux d'acétoxy-3β amino-14β étianate de méthyle (Rendement 63 %).

En reprenant les eaux-mères par de l'eau additionnée d'acide citrique et en effectuant une nouvelle extraction suivie d'une cristallisation, on obtient 0,1 g de cristaux supplémentaires, portant le rendement à 85 %.

Point de fusion F = 196-197 °C (éthanol)

Spectre IR $\nu$ = 3 430, 3 370, 3 410, 1 725, 1 250, 1 230, 1 170, 1 020 $cm^{-1}$ (Nujol) 3 360, 3 300, 1 720 (fort), 1 600, 1 260, 1 170, 1 020 $cm^{-1}$ ($CHCl_3$)

CCM ($CH_2Cl_2$—MeOH—$NH_4OH$, 96-4-0,4) Rf = 0,55

Spectre de RMN ($CDCl_3$) δ = 0,96 (3H, s) 0,98 (3H, s) 2,03 (3H, s) 0,7 à 2,3 (21H) 2,46 (1H + 2H mobiles) 3,65 (3H, s) 5,03 (1H) ppm

Exemple 6

Dihydroxy-3β, 20 amino-14β nor-21-pregnane.

On traite 0,4 g d'azide obtenu comme indiqué dans l'exemple 1, en solution dans 10 ml de tétrahydrofuranne anhydre, par 200 mg d'hydrure de lithium-aluminium ajoutés progressivement. La réaction s'effectue à température ambiante. Lorsqu'elle est complète, on hydrolyse et on noie dans 100 ml d'acétate d'éthyle. Après filtration et distillation, le résidu est lavé à l'éther puis recristallisé dans l'acétate d'éthyle.

On obtient le dihydroxy-3β, 20 amino-14β nor-21-pregnane avec un rendement de 52 % sous forme de cristaux purs.

Spectre de RMN ($CDCl_3$) δ = 0,91 (3H, s) 0,96 (3H, s) 0,7 à 2,3 (22H) 2,7 à 3,5 (4H mobiles) 3,30 (1H, d, J = 11) 3,68 (1H, d, J = 11) 4,10 (1H)

Spectre IR $\nu$ = 3 100 à 3 600, 3 390, 3 290, 2 200 à 3 100 (2 660), ppm. 1 595, 1 520, 1 110, 1 030 $cm^{-1}$ (Nujol)

Point de fusion F = 217-218 °C

Exemple 7

Par les techniques usuelles, on prépare les dérivés monoacétylé et diacétylé du dihydroxy-3β, 20-amino-14β nor-21 pregnane.

On obtient ainsi l'hydroxy-3β acétoxy-20 amino-14β nor-21 pregnane,

Point de fusion = 80 °C

Spectre IR $\nu$ = 1 725, 1 650, 1 600 $cm^{-1}$ (Nujol) l'acétoxy-3β hydroxy 20 acétamido-14β nor-21 pregnane

Point de fusion F = 232 °C

Spectre IR $\nu$ = 3 220, 1 735, 1 650, 1 575 $cm^{-1}$ (Nujol) et le diacétoxy-3β, 20 amino-14β nor-21 pregnane

Spectre IR $\nu$ = 3 400, 1 740, 1 730 $cm^{-1}$ (Nujol)

Exemple 8

Dihydroxy-3β, 17β amino-14β 5β-androstane.

On procède comme dans l'exemple 6, en remplaçant l'azide de l'exemple 1 par l'azide de l'exemple 2.

On obtient ainsi le dihydroxy-3β, 17β amino-14β 5β-androstane avec un rendement de 60 %.

Spectre IR : $\nu$ = 2 500 à 3 700, 1 590, 1 035 $cm^{-1}$ (Nujol)

CCM ($CHCl_3$—MeOH—$NH_4OH$, 90-10-1) Rf = 0,35

Par action de l'acide chlorhydrique, suivant les techniques usuelles, on prépare le chlorhydrate du dihydroxy-3β, 17β amino-14β 5β-androstane.

Point de fusion F = 260° (décomposition-Ethanol)

Spectre de RMN ($CD_3OD$) δ = 0,98 (6H, s) 0,7 à 2,4 (21H) 3,6 (1H) 4,06 (1H) ppm

## Exemple 9

Diacétoxy-3β, 17β amino-14β 5β-androstane.

On procède comme dans l'exemple 5, en remplaçant l'azide de l'exemple 1 par l'azide de l'exemple 2.

On obtient ainsi le diacétoxy-3β, 17β amino-14β 5β-androstane avec un rendement de 50 %.

Spectre IR $\nu$ = 3 000 à 3 600, 3 430, 3 385, 3 310, 3 010,

Spectre de RMN ($CDCl_3$) δ = 0,95 (6H, s) 2,03 (6H, s) 0,7 à 2,7 (21H + 2H mobiles) 5,10 (1H) 5,20 (1H) ppm

## Exemple 10

Acétoxy-3β azido-14β iso-17 étianate de méthyle.

On dissout 4g d'acétoxy-3β hydroxy-14β iso-17 étianate de méthyle, isomère du composé de départ de l'exemple 1, obtenu par la même méthode, dans 200 ml d'une solution sensiblement molaire acide azothydrique dans le benzène. On ajoute 20 ml d'éthérate de trifluorure de bore, sous agitation à température ambiante. On maintient le milieu réactionnel sous agitation pendant 15 mn.

On noie par de l'ammoniaque glacée, on lave les phases organiques par une solution de bicarbonate de sodium et on extrait au chlorure de méthylène. Après distillation on obtient un résidu blanc que l'on purifie par cristallisation dans le méthanol.

On obtient ainsi 34 g de cristaux d'acétoxy-3β azido-14β iso-17 étianate de méthyle avec un rendement de 77 %.

Point de fusion F = 157-158 °C

Spectre IR $\nu$ = 2 100, 1 730, 1 720 $cm^{-1}$ (Nujol)

Spectre de RMN ($CHCl_3$) δ = 0,96 (3H, s) 1,15 (3H, s) 2,03 (3H, s) 0,7 à 2,6 (21H) 2,7 à 3,5 (1H, m) 3,66 (3H, s) 5,06 (1H) ppm

## Exemple 11

Acétoxy-3β amino-14β iso-17 étianate de méthyle.

On procède comme indiqué dans l'exemple 5 en remplaçant l'azide de l'exemple 1 par celui de l'exemple 10.

On obtient ainsi l'acétoxy-3β amino-14β iso-17 étianate de méthyle sous forme de cristaux purs avec un rendement de 63 %.

Point de fusion F = 165-166 °C

Spectre IR $\nu$ = 3 100 à 3 600, 1 730, 1 725 $cm^{-1}$ (Nujol)

Spectre de RMN ($CDCl_3$) δ = 0,93 (3H, s) 1,10 (3H, s) 2,01 (3H, s) 0,7 à 2,5 (21H + 2H mobiles) 3,1 (1H) 3,63 (3H, s) 5,02 (1H)

## Exemple 12

Dihydroxy-3β, 20 amino-14β nor-21 (17α) pregnane

On procède comme indiqué dans l'exemple 6 en remplaçant l'azide de l'exemple 1 par celui de l'exemple 8.

On obtient ainsi, après retraitement des eaux-mères, des cristaux purs de dihydroxy-3β, 20 amino-14β nor-21 (17α) pregnane avec un rendement de 65 %.

Point de fusion F = 216-218 °C

Spectre IR $\nu$ = 2 500 à 3 600, 1 575 $cm^{-1}$ (Nujol)

Spectre de RMN ($CDCl_3$ + $CD_3OD$) δ = 0,93 (3H, s) 0,98 (3H, s) 0,7 à 2,7 (22H) 3,50 (2H) 4,05 (1H) 4,0 (4H mobiles) ppm

## Exemple 13

Amino-3ξ acétoxy-20 formamido-14β nor-21 pregnane

A une solution de 0,95 g d'hydroxy-3β acétoxy-20 amino-14β nor-21 5β-pregnane obtenu comme indiqué dans l'exemple 7, dans 10 ml d'acétone sur bain de glace, on ajoute goutte à goutte 1,6 ml de réactif de Jones (anhydride chromique dans l'acétone et l'acide sulfurique aqueux), et on laisse réagir pendant 30 mn sous agitation.

Après filtration et purification par chromatographie on obtient 0,8 g d'oxo-3 acétoxy-20 amino-14β nor-21 5β-pregnage. On dissout 0,5 g du composé obtenu comme indiqué ci-dessus et 0,1 g d'acide formique dans 10 ml de chlorure de méthylène et on ajoute goutte à goutte une solution de 0,6 g de dicyclohexylcarbodiimide dans 3 ml de chlorure de méthylène et on laisse réagir 1 heure. On obtient ainsi, après filtration 0,5 g d'oxo-3 acétoxy-20 formamido-14β nor-21 pregnane sur lequel on fait agir 0,5 g de chlorhydrate d'hydroxylamine en solution dans 10 ml d'éthanol en présence de 1 g d'acétate de sodium. Après 1 heure de réaction, on obtient ainsi l'oxime correspondante à laquelle on ajoute 5 ml de dioxanne, 1 ml d'ammoniaque (solution à 10 %), 0,2 g de chlorure d'ammonium et 6 ml d'eau. On ajoute rapidement 0,5 g d'amalgame d'aluminium. On ajoute une nouvelle fois 0,5 g d'amalgame d'aluminium après 4 heures, puis après 8 heures de réaction.

On obtient ainsi, après purification, 0,2 g d'amino-3ξ acétoxy-20 formamido-14β nor-21 pregnane.

Spectre IR = 3 130, 1 730, 1 650, 1 535 $cm^{-1}$ (Nujol)

Exemple 14

On hydrolyse 0,2 g du composé obtenu dans l'exemple 13 par 2 ml de soude 1,2N pour procurer 0,18 g de diamino-3ξ, 14β nor-21 5β-pregnanol-20 sous forme de deux isomères 3α et 3β que l'on peut séparer par chromatographie.

Chromatographie sur couche mince : Rf = 0,65 et 0,60 ($SiO_2$ ; $CHCl_3$ + MeOH + $NH_4OH$ 85-15-3).

L'isomère le moins polaire présente en RMN un singulet à 0,95 ppm ($CH_3$—18 et $CH_3$—19 confondus) et l'isomère le plus polaire présente un singulet à 0,90 ppm.

**Revendications** (pour les Etats contractants : BE, CH, DE, FR, GB, IT, LI, LU, NL, SE)

1. Dérivés d'amino-14 stéroïdes de formule générale (I)

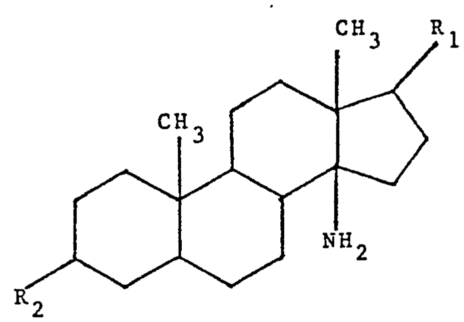

(I)

dans laquelle $R_1$ représente un atome d'hydrogène, un groupe hydroxy, hydroxyalkyle, alcoxy, acyloxy, carboxy, acyloxyalkyle, ou carbalcoxy, et $R_2$ représente un groupe hydroxy, alcoxy, acyloxy ou amino, $R_1$ ne pouvant être un groupe hydroxy-1 éthyle quand $R_2$ est un groupe hydroxy, ainsi que leurs sels d'acides.

2. Dérivés d'amino-14 stéroïdes selon la revendication 1 caractérisés en ce que $R_1$ est un groupe hydroxy, hydroxyméthyle, acétoxy, ou carbométhoxy, ou un atome d'hydrogène.

3. Dérivés d'amino-14 stéroïdes selon la revendication 1, caractérisés en ce que $R_2$ est un groupe hydroxy ou acétoxy.

4. Dérivés d'amino-14 stéroïdes selon l'une quelconque des revendications précédentes, caractérisés en ce qu'ils sont choisis parmi :

l'acétoxy-3β amino-14β étianate de méthyle
l'hydroxy-3β amino-14β androstane
le dihydroxy-3β,20 amino-14β nor-21 pregnane
l'hydroxy-3β acétoxy-20 amino-14β nor-21 pregnane
le diacétoxy-3β,20 amino-14β nor-21 pregnane
le dihydroxy-3β,17β amino-14β 5β-androstane

le diacétoxy-3β,17β amino-14β 5β-androstane
l'acétoxy-3β amino-14β iso-17 étianate de méthyle
le dihydroxy-3β,20 amino-14β nor-21 (17α) pregnane
le diamino-3,14β hydroxy-20 nor-21 5β-pregnane

5. Procédé de préparation de dérivés d'amino-14 stéroïdes de formule générale (1)

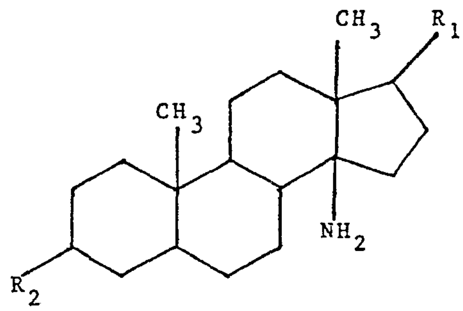

dans laquelle $R_1$ représente un atome d'hydrogène ou un groupe hydroxy, hydroxyalkyle, alcoxy, acyloxy, carboxy, acyloxyalkyle, ou carbalcoxy, et $R_2$ représente un groupe hydroxy, alcoxy ou acyloxy, $R_1$ ne pouvant pas être un groupe hydroxy-1 éthyle quand $R_2$ est un groupe hydroxy, caractérisé en ce que l'on traite un alcool de formule générale (II)

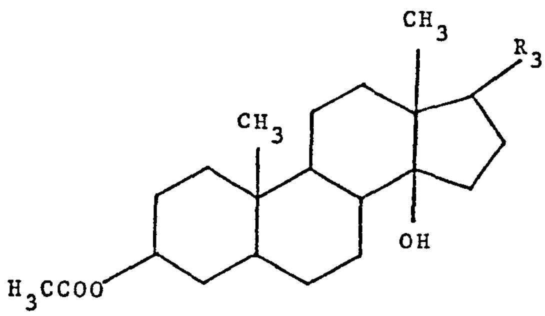

où $R_3$ est un groupe carbalcoxy ou acyloxy, ou un atome d'hydrogène, par l'acide azothydrique en présence d'éthérate de trifluorure de bore, pour former le dérivé azido-14 correspondant que l'on réduit pour former l'amino-14 stéroïde de formule (I).

6. Procédé selon la revendication 5, caractérisé en ce que l'on traite l'alcool de formule (II) par l'acide azothydrique en présence d'éthérate de trifluorure de bore, dans le benzène ou le toluène.

7. Procédé selon l'une quelconque des revendications 5 et 6, caractérisé en ce que l'acide azothydrique est utilisé en excès.

8. Procédé selon la revendication 5, caractérisé en ce que la réduction de l'azido-14 stéroïde est effectuée par hydrogénation en présence du catalyseur de Lindlar, ou par action de l'hydrure de lithium-aluminium.

9. Procédé selon l'une quelconque des revendications 5 à 8, caractérisé en ce que la réaction s'effectue à température ambiante.

**Revendications** (pour l'Etat contractant AT)

1. Procédé de préparation de dérivés d'amino-14 stéroïdes de formule générale (I)

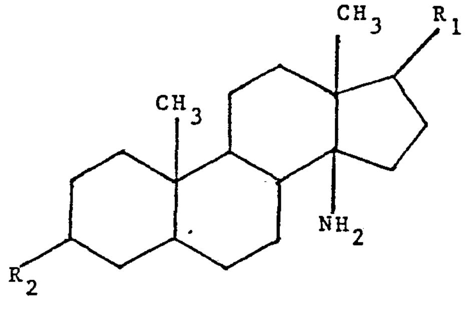

dans laquelle $R_1$ représente un atome d'hydrogène ou un groupe hydroxy, hydroxyalkyle, alcoxy, acyloxy,

carboxy, acyloxyalkyle, ou carbalcoxy, et $R_2$ représente un groupe hydroxy, alcoxy ou acyloxy, $R_1$ ne pouvant pas être un groupe hydroxy-1 éthyle quand $R_2$ est un groupe hydroxy, caractérisé en ce que l'on traite un alcool de formule générale (II)

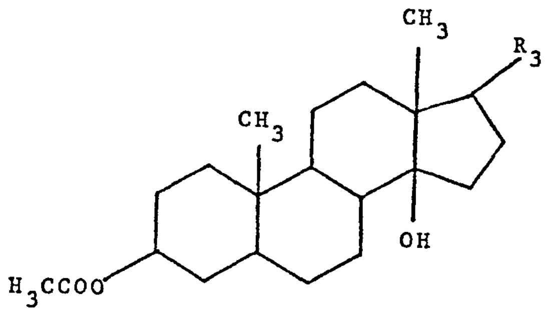

où $R_3$ est un groupe carbalcoxy ou acyloxy, ou un atome d'hydrogène, par l'acide azothydrique en présence d'éthérate de trifluorure de bore, pour former le dérivé azido-14 correspondant que l'on réduit pour former l'amino-14 stéroïde de formule (I).

2. Procédé selon la revendication 1, caractérisé en ce que l'on traite l'alcool de formule (II) par l'acide azothydrique en présence d'éthérate de trifluorure de bore, dans le benzène ou le toluène.

3. Procédé selon l'une quelconque des revendications 1 et 2, caractérisé en ce que l'acide azothydrique est utilisé en excès.

4. Procédé selon la revendication 1, caractérisé en ce que la réduction de l'azido-14 stéroïde est effectuée par hydrogénation en présence du catalyseur de Lindlar, ou par action de l'hydrure de lithium-aluminium.

5. Procédé selon l'une quelconque des revendications 1 à 4, caractérisé en ce que la réaction s'effectue à température ambiante.

**Claims** (for the Contracting States : BE, CH, DE, FR, GB, IT, LI, LU, NL, SE)

1. 14-amino steroid derivatives of the general formula (I)

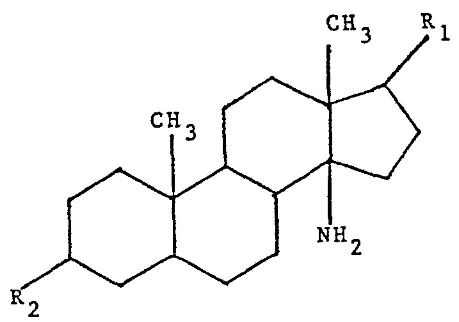

(I)

wherein $R_1$ represents a hydrogen atom, a hydroxy, hydroxyalkyl, alkoxy, acyloxy, carboxy, acyloxyalkyl or carbalkoxy group, and $R_2$ represents a hydroxy, alkoxy, acyloxy or amino group, with the proviso that $R_1$ is not a 1-hydroxyethyl group when $R_2$ is a hydroxy group, and the acid addition salts thereof.

2. The 14-amino steroid derivatives of Claim 1, characterized in that $R_1$ is a hydroxy, hydroxymethyl, acetoxy or carbomethoxy group or a hydrogen atom.

3. The 14-amino steroid derivatives of Claim 1, characterized in that $R_2$ is a hydroxy or acetoxy group.

4. The 14-amino steroid derivatives according to any of the preceding claims, characterized in that said derivatives are selected from the group consisting of :

3β-acetoxy-14β-amino-methyl-etianate,
3β-hydroxy-14β-amino-androstane,
3β,20-dihydroxy-14β-amino-21-nor-pregnane,
3β-hydroxy-20-acetoxy-14β-amino 21 nor-pregnane,
3β,20-diacetoxy-14β-amino 21-nor-pregnane,
3β-17β-dihydroxy, 14β-amino-5β-androstane,
3β,17β-diacetoxy, 14β-amino-5β-androstane,
3β-acetoxy-14β-amino-17-iso-methyl-etianate,
3β-20-dihydroxy-14β-amino-21-nor-(17α)-pregnane, and
3,14β-diamino-20-hydroxy-21-nor-5β-pregnane.

5. A process for the preparation of 14-amino steroid derivatives of the general formula (I) :

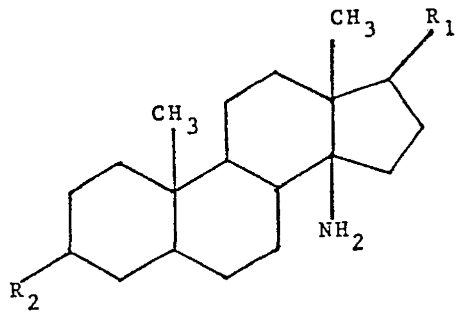

wherein $R_1$ is a hydrogen atom or a hydroxy, hydroxyalkyl, alkoxy, acyloxy, carboxy, acyloxyalkyl, or carbalkoxy group and $R_2$ is a hydroxy, alkoxy or acyloxy group, with the proviso that $R_1$ is not a 1-hydroxyethyl group when $R_2$ is a hydroxy group, characterized in that it comprises treating an alcohol of the general formula (II) :

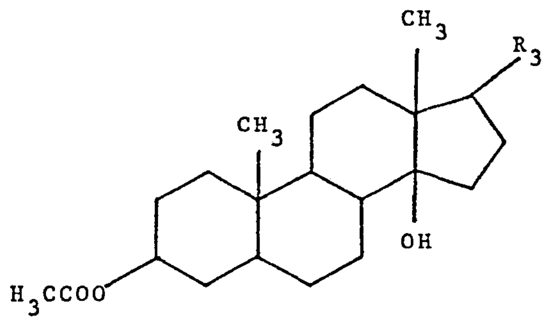

wherein $R_3$ is a carbalkoxy or acyloxy group, or a hydrogen atom, with hydrazoic acid in the presence of boron trifluoride etherate, to form the corresponding 14-azido steroid, which is reduced to form the 14-amino steroid of the general formula (I).

6. The process of Claim 5, characterized in that the treating of the alcohol of the general formula (II) with hydrazoic acid in the presence of boron trifluoride etherate is in benzene or toluene.

7. The process according to any of Claims 5 and 6, characterized in that the hydrazoic acid is used in excess.

8. The process of Claim 5, characterized in that the reducing of the 14-azido steroid is by hydrogenating the 14-azido steroid in the presence of Lindlar's catalyst, or by reducing the 14-azido steroid with lithium-aluminium hydride.

9. The process according to any of Claims 5 to 8, wherein the reaction is conducted at ambient temperature.

**Claims** (for the Contracting State AT)

1. A process for the preparation of 14-amino steroid derivatives of the general formula (I)

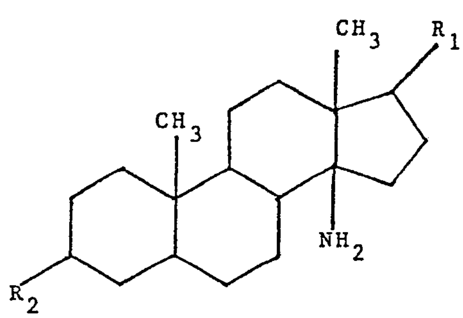

wherein $R_1$ is a hydrogen atom or a hydroxy, hydroxyalkyl, alkoxy, acyloxy, carboxy, acyloxyalkyl, or carbalkoxy group and $R_2$ is a hydroxy, alkoxy or acyloxy group, with the proviso that $R_1$ is not a 1-hydroxyethyl group when $R_2$ is a hydroxy group, characterized in that it comprises treating an alcohol of the general formula (II)

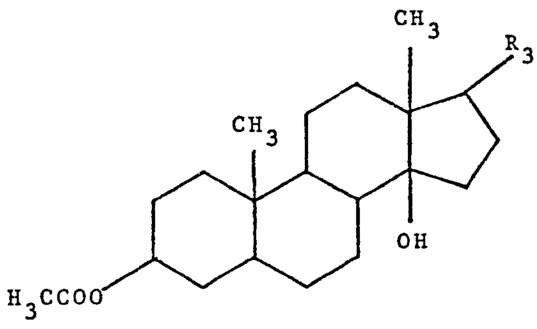

wherein $R_3$ is a carbalkoxy or acyloxy group, or a hydrogen atom, with hydrazoic acid in the presence of boron trifluoride etherate, to form the corresponding 14-azido steroid, which is reduced to form the 14-amino steroid of the general formula (I).

2. The process of Claim 1, characterized in that the treating of the alcohol of the general formula (II) with hydrazoic acid in the presence of boron trifluoride etherate is in benzene or toluene.

3. The process according to any of Claims 1 and 2, characterized in that the hydrazoic acid is used in excess.

4. The process of Claim 1, characterized in that the reducing of the 14-azido steroid is by hydrogenating the 14-azido steroid in the presence of Lindlar's catalyst, or by reducing the 14-azido steroid with lithium-aluminium hydride.

5. The process according to any of Claims 1 to 4, wherein the reaction is conducted at ambient temperature.

**Ansprüche** (für die Vertragsstaaten : BE, CH, DE, FR, GB, IT, LI, LU, NL, SE)

1. 14-Amino-steroidderivate der allgemeinen Formel (I)

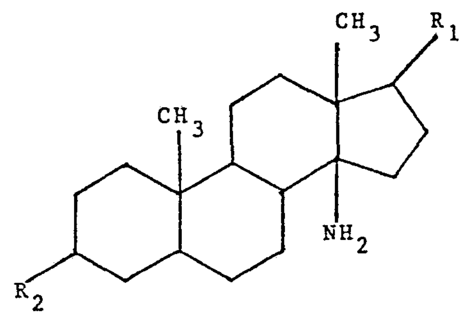

(I)

in welcher $R_1$ ein Wasserstoffatom oder eine Hydroxy-, Hydroxyalkyl-, Alkoxy-, Acyloxy-, Carboxy-, Acyloxyalkyl- oder Carbalkoxygruppe darstellt und $R_2$ eine Hydroxy-, Alkoxy-, Acyloxy- oder Aminogruppe bedeutet, wobei $R_1$ keine 1-Hydroxy-äthylgruppe sein kann, falls $R_2$ eine Hydroxygruppe ist, sowie ihre Salze mit Säuren.

2. 14-Amino-steroidderivate nach Anspruch 1, dadurch gekennzeichnet, daß $R_1$ eine Hydroxy-, Hydroxymethyl-, Acetoxy- oder Carbomethoxygruppe oder ein Wasserstoffatom darstellt.

3. 14-Amino-steroidderivate nach Anspruch 1, dadurch gekennzeichnet, daß $R_2$ eine Hydroxy- oder Acetoxygruppe ist.

4. 14-Amino-steroidderivate nach irgendeinem der vorhergehenden Ansprüche, dadurch gekennzeichnet, daß sie aus

3β-Acetoxy-14β-amino-methylätianat
3β-Hydroxy-14β-amino-androstan
3β,20-Dihydroxy-14β-amino-21-nor-pregnan
3β-Hydroxy-20-acetoxy-14β-amino-21-nor-pregnan
3β,20-Diacetoxy-14β-amino-21-nor-pregnan
3β,17β-Dihydroxy-14β-amino-5β-androstan
3β,17β-Diacetoxy-14β-amino-5β-androstan
3β-Acetoxy-14β-amino-17-iso-methylätianat
3β,20-Dihydroxy-14β-amino-21-nor(17α)-pregnan
3,14β-Diamino-20-hydroxy-21-nor-5β-pregnan

ausgewählt sind.

5. Verfahren zum Herstellen von 14-Amino-steroidderivaten der allgemeinen Formel (I)

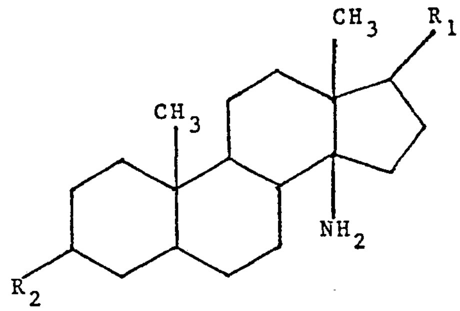

in welcher $R_1$ ein Wasserstoffatom oder eine Hydroxy-, Hydroxyalkyl-, Alkoxy-, Acyloxy-, Carboxy-, Acyloxyalkyl- oder Carbalkoxygruppe darstellt und $R_2$ eine Hydroxy-, Alkoxy- oder Acyloxygruppe bedeutet, wobei $R_1$ keine 1-Hydroxy-äthylgruppe sein kann, falls $R_2$ eine Hydroxygruppe ist, dadurch gekennzeichnet, daß ein Alkohol der allgemeinen Formel (II) :

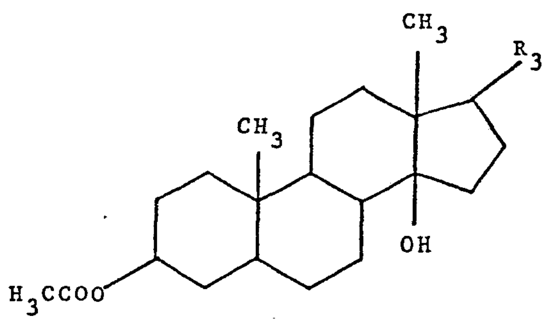

worin $R_3$ eine Carbalkoxy- oder Acyloxygruppe oder ein Wasserstoffatom darstellt, in Gegenwart des Ätherats von Bortrifluorid mit Stickstoffwasserstoffsäure behandelt und damit in das entsprechende 14-Azidoderivat übergeführt wird, welches zum 14-Amino-steroid der Formel (I) reduziert wird.

6. Verfahren nach Anspruch 5, dadurch gekennzeichnet, daß der Alkohol der Formel (II) in Benzol oder Toluol und in Anwesenheit des Ätherats von Bortrifluorid mit Stickstoffwasserstoffsäure behandelt wird.

7. Verfahren nach irgendeinem der Ansprüche 5 und 6, dadurch gekennzeichnet, daß die Stickstoffwasserstoffsäure im Überschuß angewendet wird.

8. Verfahren nach Anspruch 5, dadurch gekennzeichnet, daß die Reduktion des 14-Azidosteroids durch Hydrieren in Gegenwart eines Katalysators nach Lindlar oder mittels Lithiumaluminiumhydrid vorgenommen wird.

9. Verfahren nach irgendeinem der Ansprüche 5 bis 8, dadurch gekennzeichnet, daß die Umsetzung bei Raumtemperatur vorgenommen wird.

**Ansprüche** (für den Vertragsstaat AT)

1. Verfahren zum Herstellen von 14-Amino-steroidderivaten der allgemeinen Formel (I)

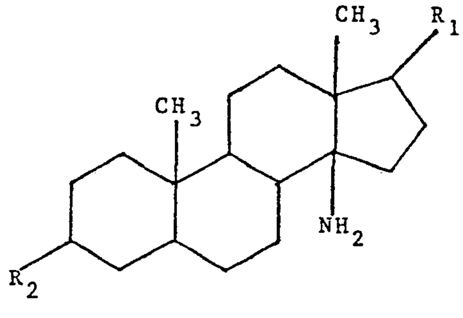

in welcher $R_1$ ein Wasserstoffatom oder eine Hydroxy-, Hydroxyalkyl-, Alkoxy-, Acyloxy-, Carboxy-, Acyloxy-alkyl- oder Carbalkoxygruppe darstellt und $R_2$ eine Hydroxy-, Alkoxy- oder Acyloxygruppe

bedeutet, wobei $R_1$ keine 1-Hydroxy-äthylgruppe sein kann, falls $R_2$ eine Hydroxygruppe ist, dadurch gekennzeichnet, dass ein Alkohol der allgemeinen Formel (II)

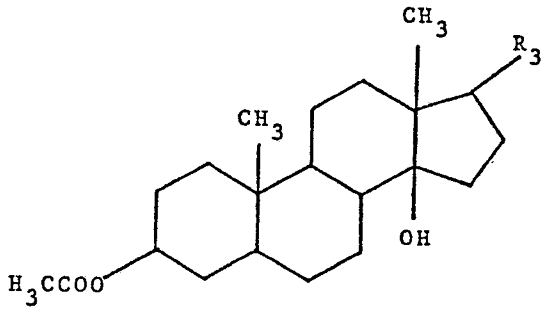

worin $R_3$ eine Carbalkoxy- oder Acyloxygruppe oder ein Wasserstoffatom darstellt, in Gegenwart des Ätherats von Bortrifluorid mit Stickstoffwasserstoffsäure behandelt und damit in das entsprechende 14-Azidoderivat übergeführt wird, welches zum 14-Amino-steroid der Formel (I) reduziert wird.

2. Verfahren nach Anspruch 1, dadurch gekennzeichnet, dass der Alkohol der Formel (II) in Benzol oder Toluol und in Anwesenheit des Ätherats von Bortrifluorid mit Stickstoffwasserstoffsäure behandelt wird.

3. Verfahren nach irgendeinem der Ansprüche 1 und 2, dadurch gekennzeichnet, dass die Stickstoffwasserstoffsäure im überschuss angewendet wird.

4. Verfahren nach Anspruch 1, dadurch gekennzeichnet, dass die Reduktion des 14-Azidosteroids durch Hydrieren in Gegenwart eines Katalysators nach Lindlar oder mittels Lithiumaluminiumhydrid vorgenommen wird.

5. Verfahren nach irgendeinem der Ansprüche 1 bis 4, dadurch gekennzeichnet, dass die Umsetzung bei Raumtemperatur vorgenommen wird.